(19) Europäisches Patentamt
European Patent Office
Office européen des brevets

(11) Publication number: **0 040 418**
**B1**

(12) **EUROPEAN PATENT SPECIFICATION**

(45) Date of publication of patent specification: **03.07.85**

(21) Application number: **81103774.6**

(22) Date of filing: **27.08.79**

(60) Publication number of the earlier application in accordance with Art. 76 EPC: **0 008 759**

(51) Int. Cl.⁴: **C 07 D 231/56** // C07C101/12, C07C103/76, C07C125/06

# (54) Process for preparing an 1H-indazol-3-ylacetic acid derivative.

(30) Priority: **12.09.78 JP 112553/78**

(43) Date of publication of application: **25.11.81 Bulletin 81/47**

(45) Publication of the grant of the patent: **03.07.85 Bulletin 85/27**

(84) Designated Contracting States: **AT BE CH DE FR GB IT LU NL SE**

(56) References cited:
**GB-A-1 517 148**

**HETEROCYCLIC COMPOUNDS, vol. 22, 1967, pages 289-305 Interscience New York, U.S.A. L.C. BEHR et al.: "Pyrazoles, pyrazolines, pyrazolidines, indazoles, and condensed rings"**

**CHEMICAL ABSTRACTS, vol. 83, no. 1, 7th July 1975, no. 10069g Columbus, Ohio, U.S.A.**

**CHEMICAL ABSTRACTS, vol. 87, no. 7, 15th August 1977, page 473, right-hand column, no. 53276x Columbus, Ohio, U.S.A.**

(73) Proprietor: **Fujisawa Pharmaceutical Co., Ltd.
3, Doshomachi 4-chome Higashi-ku
Osaka-shi, Osaka 541 (JP)**

(72) Inventor: **Kariyone, Kazuo
No. 8 Tojiinkita-machi Kita-ku
Kyoto 603 (JP)**
Inventor: **Yagi, Hideo
No. 3-27-23 Shinsenriminami-machi
Toyonaka 565 (JP)**
Inventor: **Matsushima, Hiroshi
No. 2-9-15-302 Nishishoji
Minoh-shi Osaka-prefecture 562 (JP)**

(74) Representative: **Türk, Dietmar, Dr. rer. nat. et al
Redies, Redies, Türk & Gille Patentanwälte
Brucknerstrasse 20
D-4000 Düsseldorf 13 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 040 418 B1

**Description**

This invention relates to a new process for preparing an 1H-indazol-3-ylacetic acid derivative of the formula:

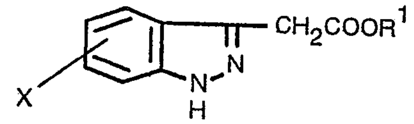

(I)

wherein $R^1$ is a hydrogen atom or a lower alkyl group, and X is a hydrogen atom or a halogen atom, or its salt.

An 1H-indazol-3-ylacetic acid derivative (I) or its salt is a known compound as a plant growth regulator and especially a sodium salt of the compound wherein X is 5-chloro and $R^1$ is hydrogen, is marketed as "RUTIACE". And, various processes for preparation thereof are also known.

From "Heterocyclic Compounds" volume 22 (1967), pages 299—300 methods of synthesizing indazoles from N-nitroso-o-toluidines and of indazoles substituted in 3-positions from ethyl 4-(2-aminophenyl)butyrate are known.

In the various processes for the preparation of the 1H-indazol-3-ylacetic acid derivative (I) or its salt, there is exemplified a method described in Japanese Patent Publication No. 82158/1975, Laid Open No. 5766/1977 as the most relevant prior art.

In said prior literature, the following methods are disclosed:

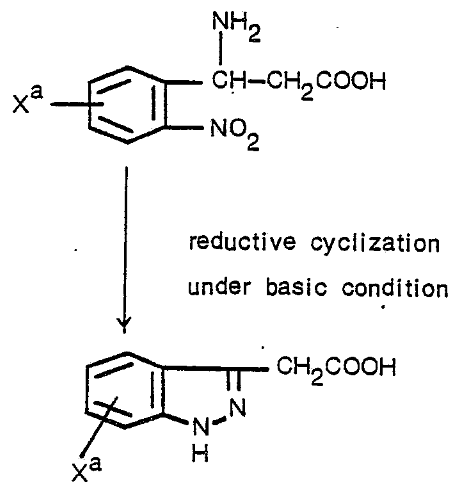


($II^a$)

($I^a$)

wherein $X^a$ is a hydrogen atom or a halogen atom.

(a) A process for preparing the 1H-indazol-3-ylacetic acid derivative ($I^a$) which comprises reacting the aminoacetic acid derivative ($II^a$) with a metal such as aluminum, zinc or the like under a basic condition.

(b) A process for preparing the 1H-indazol-3-ylacetic acid derivative ($I^a$) which comprises reacting the aminoacetic acid derivative ($II^a$) with an amalgam such as zinc amalgam, sodium amalgam or the like under a basic condition.

(c) A process for preparing the 1H-indazol-4-ylacetic acid derivative ($I^a$) which comprises subjecting the aminoacetic acid derivative ($II^a$) to a catalytic reduction in the presence of a metal catalyst such as palladium charcoal, platinum oxide, colloidal platinum or the like under basic condition.

However, the prior methods above have not led to satisfactory results in an industrial manufacture of 1H-indazol-3-ylacetic acid derivative ($I^a$) or its salt for the following reasons:

That is,

(1) In the methods (a) and (b), many complicated steps are often required for the isolation of the 1H-indazol-3-ylacetic acid derivative ($I^a$) or its salt from the reaction mixture including various metal salts. In addition it is necessary that such metal salts and amalgam are recovered and not discarded so as to prevent pollution of the environment, and furthermore the use of such an amalgam has a possibly adverse influence on working environment.

(2) The method (c) is necessarily attended with a by-product, e.g. carbostyryl derivative (6-chloro-1,2-dihydroquinolin-2-one) in the reaction mixture to that it is difficult to obtain the 1H-indazol-3-ylacetic acid derivative ($I^a$) or its salt in high yield.

As a result of extensive studies to overcoming the above mentioned problems, the present inventors have now newly found a new process for the preparation of 1H-indazol-3-ylacetic acid derivative (I) or its

salt, by which the object compound (I) or its salt can be obtained in high yield without the above mentioned various problems.

Accordingly, this invention provides a new process for preparation of 1H-indazol-3-ylacetic acid derivative (I) or its salt, by which the object compound (I) or its salt can be obtained in higher yield and in higher purity than by prior processes so that the process according to this invention is industrially advantageous.

According to this invention, 1H-indazol-3-ylacetic acid derivative (I) or its salt is prepared by the following method:

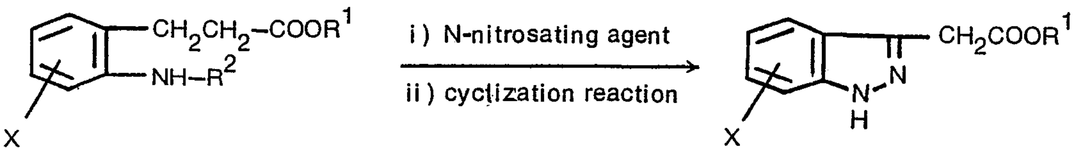


(II') (I)

wherein X is a hydrogen atom, a halogen atom, $R^1$ is a hydrogen atom or a lower alkyl group, and $R^2$ is an acyl group.

In this specification the term "lower" is intended to mean a group having 1 to 6 carbon atoms, unless otherwise indicated.

A suitable example of lower alkyl group for $R^1$ includes a straight or branched C1 to C6, preferably C1 to C4 and more preferably C1 to C2 alkyl group such as methyl, ethyl, propyl, isopropyl, butyl, isobutyl or *tert*-butyl.

The halogen atom for X includes fluorine, chlorine, bromine and iodine and preferably chlorine and bromine.

A suitable example of acyl for $R^2$ includes a lower alkanoyl (e.g. formyl, acetyl, propionyl or butyryl) a lower haloalkanoyl (e.g. chloroacetyl, dichloroacetyl or trichloroacetyl), a lower alkoxycarbonyl (e.g. methoxycarbonyl, ethoxycarbonyl, propoxycarbonyl, butoxycarbonyl isobutoxycarbonyl or *tert*-butoxycarbonyl), a lower haloalkoxycarbonyl (e.g. chloroethoxycarbonyl, dichloroethoxycarbonyl or trichloroethoxycarbonyl), an aroyl (e.g. benzoyl or toluoyl), an aralkanoyl (e.g. phenylacetyl or phenylpropionyl).

A suitable example of a salt of 1H-indazol-3-ylacetic acid derivative (I) includes a salt with an inorganic base, for example, a metal salt such as an alkali metal salt (e.g. sodium salt or potassium salt), an alkaline earth metal salt (e.g. calcium salt or magnesium salt) or an ammonium salt.

Detailed explanation of processes for preparation of 1H-indazol-3-ylacetic acid derivative (I) or its salt will be made in the following:

(1) N-nitrosation

This process is conducted by reacting the propionic acid derivative (II') or its salt with a N-nitrosating agent and then subjecting the nitrosated compound to a cyclization reaction.

The N-nitrosating agent is an agent which can be reacted with an acylamino type of nitrogen atom and suitable examples of such nitrosating agent includes nitrous acid, nitrous anhydride gas, nitrosyl chloride or alkyl nitrite (e.g. isopentyl nitrite or butyl nitrite).

This reaction is preferably conducted in an organic solvent such as acetic acid, acetic anhydride, chloroform, N,N-dimethylformamide or dimethylsulfoxide, under cooling.

(2) Cyclization reaction

The thus obtained N-nitrosated compound is then subjected to the cyclization reaction usually without being isolated from the reaction mixture.

This reaction is usually conducted in an organic solvent such as benzene, toluene, chloroform, carbon tetrachloride, diethylether, N,N-dimethylformamide or dimethylsulfoxide at ambient temperature or under warming or heating.

The thus obtained 1H-indazol-3-ylacetic acid derivative (I) or its salt of this invention is isolated from the reaction mixture in a conventional manner.

(3) Preparation of the starting compound (II')

The starting compound (II') or its salt to be used in this process is new one and can be prepared by the following methods.

(a) Preparation of the starting compound (II'a)

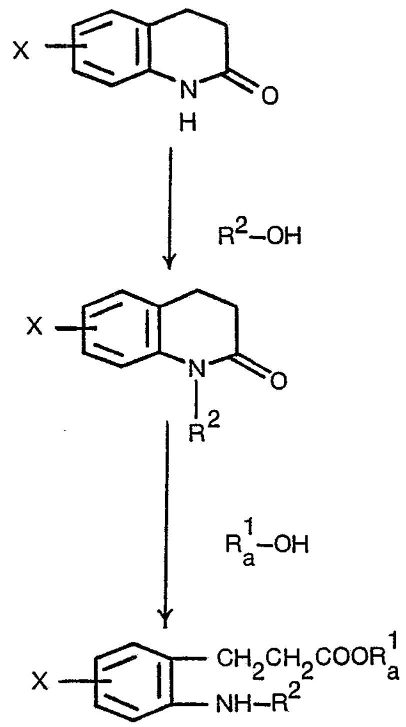


(VII)

(VIII)

(IX)

(III)

(II'a)

wherein $R_a^1$ is a lower alkyl group, and $R^2$ and X are as defined above.

Compound (VII) → Compound (IX)

This process is conducted by acylating the compound (VII) with the compound (VIII) or its salt, or its reactive derivative.

A suitable reactive derivative of the compound (VIII) includes, for example, an acid halide, an acid anhydride, an activated amide, an activated ester, and preferably acid halide such as acid chloride, acid bromide (e.g. acetyl chloride, propionyl chloride, benzoyl chloride, ethoxycarbonyl chloride).

The reaction is usually carried out in a conventional solvent such as water, acetone, dioxane, acetonitrile, chloroform, benzene, methylene chloride, ethylene chloride, tetrahydrofuran, ethyl acetate, N,N-dimethylformamide, pyridine or any other suitable solvent which does not adversely influence the reaction, or a mixture thereof.

When the compound (VIII) is used in a form of free acid or salt in this reaction, the reaction is preferably carried out in the presence of a conventional condensing agent.

This reaction is preferably carried out in the presence of a base such as an alkali metal (e.g. sodium), alkali metal or akaline earth metal hydroxide (e.g. sodium hydroxide, potassium hydroxide or calcium hydroxide).

Among the starting compounds (VII), 6-chloro-1,2,3,4-tetrahydroquinoline-2-one is known and prepared by the method described in "Chemische Berichte vol. 60, page 858—864 (1927)" and other compounds are prepared substantially in the same manner to that of the known compound.

Compound (IX) → Compound (II'a)

This process is conducted by reacting the compound (IX) with the compound (III). This reaction is usually conducted by reacting the compound (IX) with an excess amount of the compound (III), according to a process conventionally applied for so-called esterification with or without an additional other organic solvent such as diethyl ether, tetrahydrofuran, ethyl acetate, dimethylsulfoxide, N,N-dimethylformamide or any other solvent which does not adversely influence the reaction. This reaction is conducted preferably in the presence of inorganic acid (e.g. hydrochloric acid or sulfuric acid) or an organic acid (e.g. benzenesulfonic acid or trichloroacetic acid). This reaction is conducted at ambient temperature or at the boiling temperature of the solvent used in the reaction.

The thus obtained compound (II'a) is isolated from the reaction mixture in a conventional manner.

(b) Preparation of the starting compound (II') [i.e. compound (II'a) and compound (II'b)]

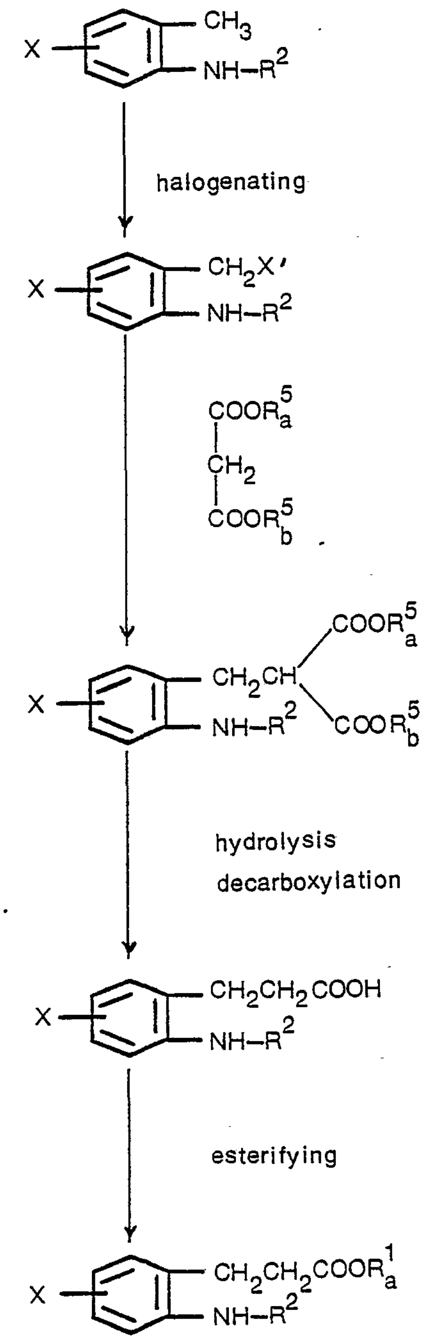


(X)

(XI)

(XII)

(XIII)

(II'b)

(II'a)

wherein $R_a^1$, $R^2$ and X are as defined above, X' is halogen, and $R_a^5$ and $R_b^5$ are lower alkyl, ar(lower)alkyl or aryl.

Compound (X) → Compound (XI)

This process is conducted by reacting the compound (X) with a halogenating agent.

The starting compounds (X) include known and new ones.

Among known compounds, ethyl N-(4-chloro-2-chloromethylphenyl)carbamate is prepared by a method described in "Journal of Medicinal Chemistry vol. 12, page 426 (1969)", and other compounds are prepared substantially in the same manner to that of the known compound.

The halogen atom for X' includes fluorine, chlorine, bromine and iodine.

This reaction is conducted by reacting the compound (X) with a halogenating agent such as sulfuryl halide (e.g. sulfuryl chloride or sulfuryl bromide).

In case that a halogen is used as a halogenating agent, the reaction is preferably initiated by irradiation with ultraviolet ray or by addition with a catalytic amount of peroxide compound (e.g. benzoylperoxide).

This reaction is usually conducted in a conventional solvent which does not adversely influence the reaction.

Compound (XI) → Compound (II'b)

This reaction is conducted by reacting the compound (XI) with an malonic acid ester (XII) to produce the compound (XIII) according to a method which is well-known and conventionally used for so-called malonic ester synthesis.

The preferred malonic acid ester of the formula (XII) is exemplified by dimethyl malonate, diethyl malonate, dipropyl malonate, methyl ethyl malonate, dibenzyl malonate and diphenethyl malonate.

The reaction is conducted in an organic solvent with a base. The base used in this reaction is exemplified by an alkali metal (e.g. sodium or potassium), an alkaline earth metal alkoxide (sodium metoxide or sodium ethoxide), or an alkaline earth metal hydroxide (e.g. sodium hydroxide or potassium hydroxide).

A suitable solvent for use in the reaction is exemplified by methanol, ethanol, tetrahydrofuran, dioxane, N,N-dimethylformamide, dimethylsulfoxide, diethylcarbonate, or a mixture thereof.

The reaction is conducted at ambient temperature or under heating.

Compound (XIII) → Compound (II'b)

This process is conducted by hydrolyzing the compound (XIII) and followed by the decarboxylation. The hydrolysis is conducted in a conventional manner such as a method using an acid (acid hydrolysis) or a base (basic hydrolysis).

The decarboxylation is conventionally conducted by heating the hydrolyzed product with or without any isolation thereof under neutral or acidic conditions. It is noted that the acidic hydrolysis may usually be accompanied with a spontaneous decarboxylation to produce the compound (II'b) or its salt from the compound (XIII) in a one-batch operation.

The compound (II'a) can be prepared by esterifying the thus obtained compound (II'b) or its salt with the compound (III) according to a conventional esterification method.

The thus obtained compounds ((II'a) and (II'b) are isolated from the reaction mixture in a conventional manner.

A suitable example of a salt of a propionic acid derivatice (II') includes the same one mentioned above as a salt of 1H-indazol-3-ylacetic acid derivative (I).

The following examples are given for illustrating the present invention in more detail.

## Example 1

### Preparation of ethyl 5-chloro-1H-indazol-3-ylacetate

(1) Preparation of ethyl 3-(2-benzamido-5-chlorophenyl)propionate

Benzoyl chloride (3.0 g) was added dropwise to a solution of 6-chloro-1,2,3,4-tetrahydroquinolin-2-one (2.0 g) in pyridine (40 ml) with stirring. The mixture was heated under reflux at 140—150°C for 3 hours. The resultant solution was concentrated under reduced pressure. The residue was dissolved in chloroform (10 ml) and washed with 5% aqueous hydrochloric acid solution (10 ml). The organic layer was separated, dried over anhydrous sodium sulfate, and concentrated under reduced pressure. The residue was dissolved in ethanol (70 ml) saturated with dry hydrogen chloride and heated under reflux for 1 hour. The resultant solution was evaporated under reduced pressure and the residue was dissolved in a mixture of benzene and n-hexane (1:2) and crystallized to give colorless crystals of ethyl 3-(2-benzamido-5-chlorophenyl)propionate (2.7 g). M.p. 105—106°C.

N.M.R. ($CCl_4$) δ (ppm): 10.60 (1H, s), 8.1—7.7 (2H, m), 7.68 (1H, d, J=9Hz), 7.6—7.1 (3H, m), 7.08 (1H, dd, J=2Hz, J=9Hz), 7.04 (1H, d, J=2Hz), 4.03 (2H, q, J=7Hz), 2.4—3.1 (4H, m), 1.14 (3H, t, J=7Hz).

(2) Preparation of ethyl 5-chloro-1H-indazol-3-ylacetate

Ethyl 3-(2-benzamido-5-chlorophenyl)propionate (1.5 g) was suspended in a mixture of acetic anhydride (2 ml) and acetic acid (2 ml). Nitrous anhydride gas which was generated by dropwise addition of nitric acid (d.l. 47, 4 ml) to sodium nitrite (4 g) was introduced into the suspension at 0—5°C for 15 minutes, and then the resulting dark green solution was stirred at the same temperature for 40 minutes. The solution was poured onto ice (50 g) and stirred at ambient temperature for 1 hour. The resultant mixture was extracted with benzene (50 ml) and washed with two portions of water (10 ml). To the extract was added methanol (3 ml) and the resulting solution was left to stand at ambient temperature for 2 hours. Then, the solution was washed with water (2 times), dried over calcium chloride and filtered. The filtrate was heated under reflux for 7 hours and evaporated to dryness to give oily residue. The residue was dissolved in ethanol, treated with activated charcoal and concentrated under reduced pressure. The residue was crystallized from diisopropyl ether. The obtained crystals were recrystallized from diisopropyl ether to give colorless crystals of ethyl 5-chloro-1H-indazol-3-ylacetate (700 mg). M.p. 76—77°C.

Example 2

Preparation of methyl 5-chloro-1H-indazol-3-ylacetate

(1) Preparation of ethyl N-(4-chloro-2-chloromethylphenyl)carbamate

Ethyl N-(4-chloro-2-methylphenyl)carbamate (40 g) was suspended in a solution of chlorine (18.5 g) in dry carbon tetrachloride (250 ml). The suspension was stirred under the irradiation of ultraviolet-ray liberated from a high-pressure mercury lamp at ambient temperature for 1 hour. The product which was precipitated in the reaction mixture was collected by filtration and crystallized from a mixture of ethyl acetate and n-hexane to give ethyl N-(4-chloro-2-chloromethylphenyl)carbamate (29 g). M.p. 137—139°C.

Analysis
Calculated for $C_{10}H_{11}NCl_2O_2$:
C 48.41%, H 4.47%, N 5.65%, Cl 28.58%
found:
C 48.66%, H 4.4%, N 5.64%, Cl 28.62%

(2) Preparation of Diethyl (5-chloro-2-ethoxycarbonylaminobenzyl)malonate

Ethyl N-(4-chloro-2-chloromethylphenyl)carbamate (28.5 g) was added to a solution of diethyl malonate (29 g) and sodium hydroxide (6.5 g) in ethanol (300 ml). The mixture was stirred at ambient temperature for 15 hours. The resultant solution was evaporated under reduced pressure. The residue was dissolved in benzene (300 ml), washed with water, dried over anhydrous sodium sulfate, filtered and concentrated under reduced pressure. The residue was crystallized from benzene and recrystallized from diisopropyl ether to give colorless needles of diethyl (5-chloro-2-ethoxycarbonylaminobenzyl)-malonate (15.0 g). M.p. 86—88°C.

(3) Preparation of (5-chloro-2-ethoxycarbonylaminobenzyl)malonic acid

Diethyl (5-chloro-2-ethoxycarbonylaminobenzyl)malonate (19 g) and barium hydroxide [$Ba(OH)_2 \cdot 8H_2O$] (19 g) were suspended in a mixture of methanol (100 ml) and water (50 ml). The suspension was stirred at ambient temperature for 30 minutes to give precipitates. The precipitates were collected by filtration and suspended in water. The suspension was acidified to pH 1.0 with hydrochloric acid and extracted with ethyl acetate. The organic layer was washed with water, dried over anhydrous sulfate, filtered and evaporated under reduced pressure. The residue was crystallized from the mixture of ethyl acetate and benzene to give colorless crystals of (5-chloro-2-ethoxycarbonylaminobenzyl)malonic acid (14.4 g). M.P. 133—136°C (dec.).

N.M.R. (DMSO-$d_6$) δ (ppm) 8.95 (1H, s), 7.2 (3H, m), 4.13 (2H, q, J=7Hz), 3.62 (1H, t, J=8Hz), 3.10 (2H, d, J=8Hz), 1.25 (3H, t, J=7Hz).

(4) Preparation of 3-(5-chloro-2-ethoxycarbonylaminophenyl)propionic acid

The crystals of (5-chloro-2-ethoxycarbonylaminobenzyl)malonic acid (14.4 g) were heated at 140—145°C on oil bath for 40 minutes. The resulting crystallized mass, after cooling, was dissolved in 5% aqueous sodium bicarbonate solution (150 ml) and the solution was washed with ethyl acetate and acidified to pH 1 with hydrochloric acid to precipitate crystals. The crystals were separated by filtration, washed with water and dissolved in hot ethanol. The solution was treated with activated charcoal and filtered. Addition of water to the filtrate gave colorless needles of 3-(5-chloro-2-ethoxycarbonylamino-phenyl)propionic acid (11.1 g). M.p. 186—187°C.

N.M.R. (DMSO-$d_6$) δ (ppm) 8.96 (1H, s), 7.3 (3H, m), 4.12 (2H, q, J=6.5Hz), 2.67 (4H, m), 1.22 (3H, t, J=6.5Hz).

(5) Preparation of methyl 3-(5-chloro-2-ethoxycarbonylaminophenyl)propionate

3-(5-Chloro-2-ethoxycarbonylaminophenyl)propionic acid (9.5 g) was dissolved in a mixture of dry methanol (200 ml) and sulfuric acid (0.5 g) and stirred at ambient temperature for 24 hours. After neutralization with triethylamine (1 g) the resultant solution was concentrated under reduced pressure. The oily residue was dissolved in ethyl acetate (100 ml), washed with 5% aqueous sodium bicarbonate solution dried over anhydrous sodium sulfate, filtered and evaporated under reduced pressure. The residue was crystallized from a mixture of benzene and n-hexane to give colorless needles of methyl 3-(5-chloro-2-ethoxycarbonylaminophenyl)propionate (10 g). M.p. 70—72°C.

Analysis
Calculated for $C_{13}H_{16}NO_4Cl$:
C 54.65%, H 5.64%, N 4.90%, Cl 12.41%
found:
C 54.67%, H 5.65%, N 4.78%, Cl 12.43%

(6) Preparation of methyl 5-chloro-1H-indazol-3-ylacetate

Methyl 3-(5-chloro-2-ethoxycarbonylaminophenyl)propionate (5 g) was suspended in a mixture of

acetic anhydride (6 ml) and acetic acid (8 ml). Nitrous anhydride gas which was generated by dropwise addition of nitric acid (d. I.47,7 ml) to sodium nitrite (5 g) was introduced into the suspension at 5°C for 1.5 hours, and then the mixture was stirred at ambient temperature for 30 minutes. The resultant dark green solution was poured onto ice (100 g) and stirred at the same temperature for 1 hour. The resultant mixture was extracted with two portions of benzene (70 ml). The combined extracts were added to methanol (5 ml) and left to stand at ambient temperature for 1 hour. Then, the solution was washed with water (2 times), dried over anhydrous sodium sulfate and filtered. The filtrate was heated under reflux for 16 hours and evaporated to dryness to give an oily residue. The residue was dissolved in methanol, treated with activated charcoal and concentrated under reduced pressure. The residue was crystallized from diisopropyl ether. The obtained crystals were recrystallized from diisopropyl ether to give colorless needles of methyl 5-chloro-1H-indazol-3-ylacetate (2.6 g). M.p: 87—88°C.

N.M.R. ($CDCl_3$) δ (ppm): 7.64 (1H, d, J=2Hz), 7.23 (2H, m), 4.00 (2H, s), 3.72 (3H, s).

## Claims

1. A process for preparing an 1H-indazol-3-ylacetic acid derivative of the formula:

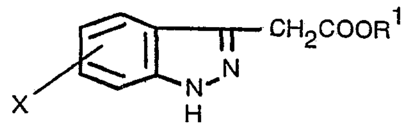

wherein $R^1$ is a hydrogen atom or a $C_1$—$C_6$- alkyl group, and X is a hydrogen atom, or a halogen atom, or its salt which is characterized by reacting a propionic acid derivative of the formula:

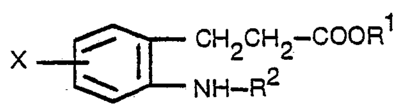

wherein $R^1$ and X are as defined above, and $R^2$ is an acyl group, or its salt with an N-nitrosating agent, and then subjecting the thus obtained compound to cyclization to give the said compound of the formula:

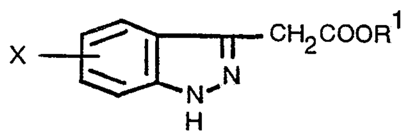

wherein $R^1$ and X is as defined above or its salt.

2. A process of claim 1, wherein the reaction of cyclization is conducted under warming or heating.

3. A process of claim 1, wherein $R^1$ is methyl and X is 5-chlorine.

4. A process of claim 2, wherein $R^1$ is methyl and X is 5-chlorine.

5. A process of claim 1, wherein $R^1$ is ethyl and X is 5-chlorine.

6. A process of claim 2, wherein $R^1$ is ethyl and X is 5-chlorine.

## Patentansprüche

1. Verfahren zur Herstellung eines 1H-Indazol-3-yl-essigsäure-Derivats der Formel:

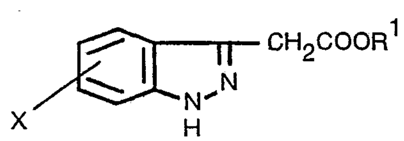

worin $R^1$ ein Wasserstoffatom oder eine $C_1$—$C_6$-Alkylgruppe ist und X ein Wasserstoffatom oder ein Halogenatom ist, oder eines Salzes, dadurch gekennzeichnet, daß man ein Propionsäure-Derivat der Formel:

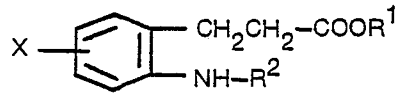

worin $R^1$ und X wie oben definiert sind und $R^2$ eine Acylgruppe ist, oder sein Salz mit einem N-nitrosierenden Mittel umsetzt und dann die so erhaltene Verbindung der Zyklisierung zur Bildung der Verbindung der Formel:

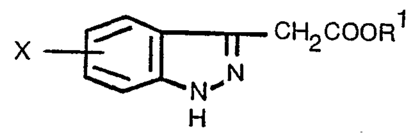

worin $R^1$ und X wie oben definiert sind, oder ihres Salzes umsetzt.

2. Verfahren gemäß Anspruch 1, worin die Zyklisierungsreaktion unter Erwärmen oder Erhitzen durchgeführt wird.

3. Verfahren gemäß Anspruch 1, worin $R^1$ Methyl ist und X 5-Chlor ist.

4. Verfahren gemäß Anspruch 2, worin $R^1$ Methyl ist und X 5-Chlor ist.

5. Verfahren gemäß Anspruch 1, worin $R^1$ Ethyl ist und X 5-Chlor ist.

6. Verfahren gemäß Anspruch 2, worin $R^1$ Ethyl ist und X 5-Chlor ist.

**Revendications**

1. Procédé de préparation d'un dérivé de l'acide 1H-indazol-3-ylacétique répondant à la formule

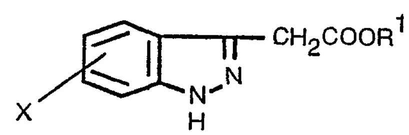

dans laquelle $R^1$ représente un atome d'hydrogène ou un groupe alkyle en $C_1$—$C_6$ et X représente un atome d'hydrogène ou un atome d'halogène, ou d'un de ses sels, caractérisé en ce qu'on fait réagir un dérivé d'acide propionique répondant à la formule:

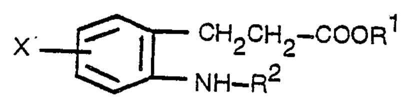

dans laquelle $R^1$ et X sont tels que définis ci-dessus, et $R^2$ représente un groupe acyle, ou un de ses sels, avec un agent N-nitrosant, et en ce qu'on soumet ensuite le composé ainsi obtenu à une cyclisation pour obtenir le composé répondant à la formule

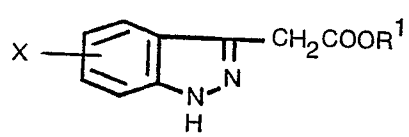

dans laquelle $R^1$ et X sont tels que définis ci-dessus, ou un de ses sels.

2. Procédé selon la revendication 1, caractérisé en ce qu'on met en oeuvre la réaction de cyclisation en chauffant.

3. Procédé selon la revendication 1, caractérisé en ce que $R^1$ représente le groupe méthyle et X le groupe 5-chloro.

4. Procédé selon la revendication 2, caractérisé en ce que $R^1$ représente le groupe méthyle et X le groupe 5-chloro.

5. Procédé selon la revendication 1, caractérisé en ce que $R^1$ représente le groupe éthyle et X le groupe 5-chloro.

6. Procédé selon la revendication 2, caractérisé en ce que $R^1$ représente le groupe éthyle et X le groupe 5-chloro.